(19) Europäisches Patentamt
European Patent Office
Office européen des brevets

(11) Veröffentlichungsnummer: **0 384 370 B1**

(12) # EUROPÄISCHE PATENTSCHRIFT

(45) Veröffentlichungstag der Patentschrift: **03.11.93**

(51) Int. Cl.[5]: **C07D 239/42**, A61K 49/00

(21) Anmeldenummer: **90103186.4**

(22) Anmeldetag: **20.02.90**

(54) **Substituierte Pyrimidin-Derivate, Verfahren zu ihrer Herstellung und ihre Verwendung als Tool.**

(30) Priorität: **22.02.89 DE 3905364**

(43) Veröffentlichungstag der Anmeldung:
**29.08.90 Patentblatt 90/35**

(45) Bekanntmachung des Hinweises auf die Patenterteilung:
**03.11.93 Patentblatt 93/44**

(84) Benannte Vertragsstaaten:
**AT BE CH DE DK ES FR GB GR IT LI LU NL SE**

(56) Entgegenhaltungen:

| | |
|---|---|
| **EP-A- 0 323 757** | **EP-A- 0 330 263** |
| **DE-A- 1 695 975** | **DE-A- 2 152 742** |
| **DE-A- 2 263 052** | **DE-A- 2 520 381** |
| **DE-B- 2 149 249** | **DE-B- 2 433 176** |
| **GB-A- 959 699** | **GB-A- 2 198 132** |

**JOURNAL OF THE CHEMICAL SOCIETY, CHEMICAL COMMUNICATIONS, Nr. 24, 1976, Seiten 1060-1061; J.H. FORSBERG et al.: "Infrared spectroscopic evidence for the formation of an intermediate involved in the Zeolite-catalysed of n-butenes upon addition of sulphur dioxide"**

**JOURNAL OF ORGANIC CHEMISTRY, Band 52, 1987, Seiten 1017-1021; J.H. FORSBERG et al.: "Use of Lanthanide (III) ions as catalysts for the reactions of amines with nitriles"**

(73) Patentinhaber: **HOECHST AKTIENGESELLSCHAFT**

**D-65926 Frankfurt(DE)**

(72) Erfinder: **Geisen, Karl, Dr.**
**Jahnstrasse 43**
**D-6000 Frankfurt am Main(DE)**
Erfinder: **Lang, Hans-Jochen, Dr.**
**Rüdesheimer Strasse 7**
**D-6238 Hofheim am Taunus(DE)**
Erfinder: **Nimmesgern, Hildegard, Dr.**
**Rauenthaler Weg 32**
**D-6000 Frankfurt am Main 71(DE)**
Erfinder: **Weidmann, Klaus, Dr.**
**Talweg 11**
**D-6242 Kronberg/Taunus(DE)**

**EP 0 384 370 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Erhöhte intracelluläre Sorbit-Konzentrationen werden als Ursache diabetischer Spätschäden wie z.B. der Retinopathie, Neuropathie und Nephropathie angesehen. Sorbit wird durch das Enzym-Aldose-Reduktase bei erhöhter Blutglucose vermehrt gebildet. Die Sorbit-Akkumulation kann durch Aldose-Reduktase-Hemmer verhindert werden.

Das Screening nach Aldose-Reductase Inhibitoren (ARI) erfolgt an Streptozotocin-diabetischen Ratten 1 bis 2 Wochen nach Diabetesinduktion mit 60 mg Streptozotocin-Sulfat pro kg/Ratte werden die Tiere im ARI-Screening eingesetzt. Als Maß der Wirksamkeit von Aldose-Reductase-Inhibitoren dient die Absenkung des erhöhten Sorbitolgehalts in Erythrozyten, in Nerven und der Linse 5 - 6 h nach Behandlung mit den zu untersuchenden ARIs.

Streptozotocin ist ein Cancerogen. Applikation von Streptozotocin und Haltung der Tiere nach Applikaiton (2-3 Tage) müssen daher unter Biohazard-Bedingungen erfolgen. Der während der ersten 2 Tage nach Streptozotocin-Applikation ausgeschiedene Urin muß besonders entsorgt werden, die kontaminierten Boxen speziell gereinigt werden. Streptozotocin wirkt aber nicht nur cancerogen und Betazell-toxisch, es verursacht auch Leber- und Nierenschäden. Deshalb werden die Tiere erst 10 - 14 Tage nach Applikation im ARI-Screening eingesetzt.

Es wurde nun überraschenderweise gefunden, daß Pyrimidin-Derivate der Formel I

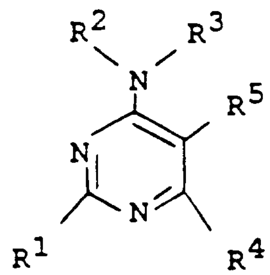

I

und deren pharmakologisch verträgliche Salze ohne Einfluß auf die Blutglukose akut und chronisch, oral oder parenteral verabreicht einen intracellulären Sorbitanstieg verursachen. Der durch die Pyrimidin-Derivate der Formel I induzierte Sorbitanstieg wird durch simultane Behandlung mit Aldose-Reductase-Inhibitoren verhindert. Die Sorbit-akkumulierenden Pyrimidin-Derivate eignen sich daher für ein neues, vereinfachtes, weniger kosten- und zeitaufwendiges Akut-Screening nach Aldose-Reductase-Inhibitoren an normalen, nicht diabetischen Ratten.

Durch Induktion von funktionellen und morphologischen Veränderungen im Sinne diabetischer Spätschäden bei chronisch mit Pyrimidin-Derivaten der Formel I behandelten Tieren, z. B. durch Verabreichung im Trinkwasser, kann auch gezeigt werden, daß die intracelluläre Sorbit-Akkumulation tatsächlich die direkte Ursache der diabetischen Spätschäden ist.

Parameter für diabetische Spätschäden sind:

Nervenleitgeschwindigkeit, Pupillendilatation, Retina-Capillar-Aneurysmen, Dicke der Basalmembran der Capillaren.

Die vorliegende Erfindung betrifft daher Pyrimidin-Derivate der Formel I

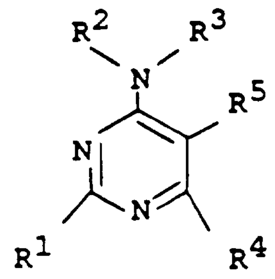

I

in welcher

$R^1$ und $R^5$ gleich sind oder verschieden sind und Wasserstoff oder ($C_1$-$C_6$)-Alkyl bedeuten, $R^4$ Wasserstoff ist,

$R^2$, $R^3$ zusammen mit dem Stickstoff, an den sie gebunden sind, die Piperazinogruppe bilden oder eine mit gleichen oder verschiedenen Gruppen $R^6$ und $R^7$ substituierte Piperazinogruppe darstellen, wobei $R^6$, $R^7$ ($C_1$-$C_6$)-Alkyl, Sulfamoyl, N-($C_1$-$C_4$)-Alkylsulfamoyl, N,N-($C_1$-$C_4$)-Dialkylsulfamoyl, ($C_1$-$C_6$)-Alkoxycarbonyl, N,N-($C_1$-$C_4$)-Dialkylcarbamoyl, N-($C_1$-$C_4$)-Alkylcarbamoyl, N-($C_6$-$C_{12}$)-Arylcarbamoyl, im Arylrest mit ($C_1$-$C_4$)-Alkyl, ($C_1$-$C_4$)-Alkoxy, Halogen, $NO_2$, $NH_2$, CN oder $CF_3$ substituiertes ($C_6$-$C_{12}$)-Arylcarbamoyl, Carbamoyl, ($C_1$-$C_6$)-Alkylcarbonyl, ($C_6$-$C_{12}$)-Arylcarbonyl, im Arylrest mit ($C_1$-$C_4$)-Alkyl, ($C_1$-$C_4$)-Alkoxy, Halogen, $NO_2$, $NH_2$, CN oder $CF_3$ substituiertes ($C_6$-$C_{12}$)-Arylcarbonyl, ($C_1$-$C_6$)-Alkylsulfonyl, ($C_1$-$C_6$)-Alkylsulfinyl, ($C_6$-$C_{12}$)-Arylsulfonyl, im Arylrest mit ($C_1$-$C_4$)-Alkyl, ($C_1$-$C_4$)-Alkoxy, Halogen, $NO_2$, $NH_2$, CN oder $CF_3$ substituiertes ($C_6$-$C_{12}$)-Arylsulfonyl, unsubstituiertes Heteroarylcarbonyl oder Heteroarylsulfonyl, wobei die Heteroarylreste ein Sauerstoffatom oder 1 bis 3 Stickstoffatome aufweiser, bedeuten oder einer der Substituenten $R^6$, $R^7$ Wasserstoff ist, sowie deren physiologisch verträgliche Salze.

In den vorstehenden und folgenden Definitionen stehen Alkyl und Alkoxy (auch in abgeleiteten Resten) für geradkettige oder verzweigte Reste, Halogen steht für Fluor, Chlor, Brom und Jod, insbesondere für Chlor.

($C_6$-$C_{12}$)-Aryl ist beispielsweise Phenyl, Naphthyl oder Biphenyl.

Bevorzugt sind Pyrimidin-Derivate der Formel I, worin $R^1$, $R^4$ und $R^5$ die angegebenen Bedeutungen haben, und

$R^2$, $R^3$ zusammen mit dem Stickstoff, an den sie gebundenen sind, einen Piperazin-Ring bilden, der gegebenenfalls durch gleiche oder verschiedene Gruppen $R_6$ und $R^7$ substituiert ist, wobei

$R^6$, $R^7$ ($C_1$-$C_6$)-Alkyl, Sufamoyl, N-($C_1$-$C_4$)-Alkylsulfamoyl, N,N-($C_1$-$C_4$)-Dialkylsulfamoyl, ($C_1$-$C_6$)-Alkoxycarbonyl, N,N-($C_1$-$C_4$)-Dialkylcarbamoyl, N-($C_1$-$C_4$)-Alkylcarbamoyl, Carbamoyl, ($C_1$-$C_6$)-Alkylcarbonyl, ($C_6$-$C_{12}$)-Arylcarbonyl, im Arylrest mit ($C_1$-$C_4$)-Alkyl, ($C_1$-$C_4$)-Alkoxy, Halogen, $NO_2$, $NH_2$, CN oder $CF_3$ substituiertes ($C_6$-$C_{12}$)Arylcarbonyl, ($C_1$-$C_6$)-Alkylsulfonyl, ($C_1$-$C_6$)-Alkylsulfinyl, ($C_6$-$C_{12}$)-Arylsulfonyl, im Arylrest mit ($C_1$-$C_4$)-Alkyl, ($C_1$-$C_4$)Alkoxy, Halogen, $NO_2$, $NH_2$, CN oder $CF_3$ substituiertes ($C_6$-$C_{12}$)-Arylsulfonyl, unsubstituiertes Heteroarylcarbonyl oder Heteroarylsulfonyl, wobei die Heteroarylreste ein Sauerstoffatom oder 1 bis 3 Stickstoffatome aufweisen, bedeuten oder einer der Substituenten $R^6$, $R^7$ Wasserstoff ist, sowie deren physiologisch verträgliche Salze.

Besonders bevorzugt sind Pyrimidin-Derivate der Formel I, worin

$R^1$ und $R^5$ gleich oder verschieden sind und Wasserstoff oder ($C_1$-$C_4$)-Alkyl bedeuten, $R^4$ Wasserstoff ist und

$R^2$, $R^3$ zusammen mit dem Stickstoff, an den sie gebunden sind, einen Piperazin-Ring bilden, der gegebenenfalls in Position 4 einen weiteren Substituenten $R^6$ trägt, wobei

$R^6$ Sulfamoyl, N-($C_1$-$C_4$)-Alkylsulfamoyl, N,N-($C_1$-$C_4$)Dialkylsulfamoyl, Carbamoyl, N-($C_1$-$C_4$)-Alkylcarbamoyl, N,N-($C_1$-$C_4$)-Dialkylcarbamoyl, ($C_1$-$C_6$)-Alkylcarbonyl, ($C_6$-$C_{12}$)-Arylcarbonyl, im Arylrest mit ($C_1$-$C_4$)-Alkyl, ($C_1$-$C_4$)-Alkoxy, Halogen, $NO_2$, $NH_2$, CN oder $CF_3$ substituiertes ($C_6$-$C_{12}$)-Arylcarbonyl oder Pyridincarbonyl bedeutet, sowie deren physiologisch verträgliche Salze.

Ganz besonders bevorzugt sind Pyrimidin-Derivate der Formel I, worin

$R^1$ Wasserstoff oder ($C_1$-$C_2$)-Alkyl, insbesondere Methyl, bedeutet,

$R^4$ Wasserstoff bedeutet

$R^5$ Wasserstoff ist

$R^2$, $R^3$ zusammen mit dem Stickstoff, an den sie gebunden sind, einen Piperazin-Ring bilden, der gegebenenfalls in Position 4 einen weiteren Substitueten $R^6$ trägt, wobei $R^6$ N-($C_1$-$C_3$)-Alkylsulfamoyl, N,N-($C_1$-$C_2$)-Dialkylsulfamoyl, N-($C_1$-$C_2$)-Alkylcarbamoyl, N,N-($C_1$-$C_2$)-Dialkylcarbamoyl, ($C_1$-$C_2$)-Alkylcarbonyl, Phenylcarbonyl, das gegebenenfalls im Phenylrest substituiert ist mit ($C_1$-$C_2$)-Alkyl, Chlor oder $NO_2$, oder Pyridincarbonyl ist, insbesondere N,N-Dimethylsulfamoyl, Phenylcarbonyl oder Pyridincarbonyl darstellt, sowie deren physiologisch verträgliche Salze.

Die Erfindung betrifft weiterhin ein Verfahren zur Herstellung von Verbindungen der Formel I, das dadurch gekennzeichnet ist, daß man in an sich bekannter Weise

a) eine Verbindung der Formel II

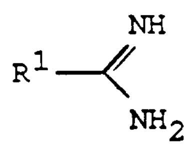

II

worin $R^1$ die zu Formel I angegebenen Bedeutungen hat oder deren Säureadditionssalz, mit einer Verbindung der Formel III

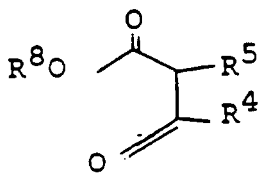

III

worin $R^4$ und $R^5$ die zu Formel I angegebenen Bedeutungen haben und $R^8$ Methyl oder Ethyl bedeutet, oder mit deren Basensalz zu einer Verbindung der Formel IV

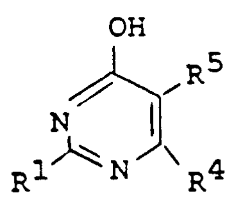

IV

worin $R^1$, $R^4$ und $R^5$ die zur Formel I angegebenen Bedeutungen hat, umsetzt

b) eine erhaltene Verbindung IV mit einem anorganischen Säurechlorid, wie z. B. mit Phosphoroxychlorid, zu einem Pyrimidin-Derivat der Formel V

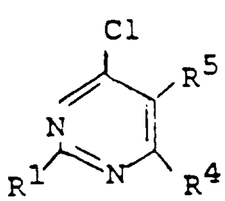

V

in welcher die Reste $R^1$, $R^4$ und $R^5$ die zu Formel I angegebenen Bedeutungen haben, umsetzt,

c) eine erhaltene Verbindung der Formel V mit einem Amin der Formel VI

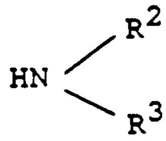

VI

in der $R^2$ und $R^3$ die zu Formel I angegebenen Bedeutungen hat, zu einer Verbindung der Formel I umsetzt, und

d) gegebenenfalls in einer erhaltenen Verbindung der Formel I, worin $R^2$ und $R^3$ gemeinsam mit dem sie tragenden Stickstoffatom den Piperazinorest bilden, den oder die Reste $R^6$/$R^7$ einführt, und

e) gegebenenfalls eine erhaltene Verbindung der Formel I in ein physiologisch verträgliches Salz überführt.

Das erfindungsgemäße Verfahren wird analog den in der Literatur beschriebenen Verfahren durchgeführt (vergl. z.B. D.J. Brown, The Chemistry of Heterocyclic Compounds, The Pyrimidines Suppl. I (1970), Suppl. II (1985), Wiley-Interscience, N.Y. und darin zitierte Literatur).

Durch Umsetzung von Verbindungen der Formel V mit Aminen der Formel VI, in denen $R^2$, $R^3$ zusammen mit dem Stickstoff, an den sie gebunden sind, einen Piperazinrest bilden, kommt man zu Verbindungen der Formel I, in denen entweder das Ringsystem schon die Substituenten $R^6$, $R^7$ wie oben definiert wurde, trägt oder unsubstituiert ist. Dieses Piperazinringsystem trägt noch aoide Wasserstoffatome, die gegebenenfalls durch Umsetzung mit Verbindungen Z-$R^6$/Z-$R^7$, worin Z Chlor, Brom oder Jod bedeutet und $R^6$/$R^7$ die zu Formel I angegebenen Bedeutungen hat, substituiert werden.

Verbindungen der Formel I können durch Umsetzung mit Säuren in ihre physiologisch verträglichen Salze überführt werden.

Die erfindungsgemäßen Verbindungen provozieren durch eine intrazelluläre Polyol-Akkumulation ohne diabetische Stoffwechsellage funktionelle Symptome im Sinne einer diabetischen Neuropathie.

Pharmakologische Untersuchung

In Dosen von 5 - 50 mg/kg Ratte oral verabreicht, verursachten die Verbindungen gemäß der vorliegenden Erfindung einen von der Dosis abhängigen Anstieg der Sorbitolkonzentration im Ichidiacus-Nerv und in den Erythrocyten normaler und Streptozotocin-diabetischer Ratten innerhalb von 4 bis 5 Stunden.

Nach oraler Verabreichung von 25 mg/kg Ratte der Verbindung gemäß Beispiel 1d wird nach 4 - 5 Stunden bei normalen Ratten eine Sorbitolkonzentration in den genannten Geweben erreicht, die derjenigen entspricht, welche Streptozotocindiabetische Ratten nach 8 Tagen aufweisen. Durch simultane orale Behandlung mit dem ARI Spiro-2,7-difluor-9H-fluoren-9,4-imidazolidin-2,5-dion (= HOE 843) wird der Sorbitolanstieg dosisabhängig verhindert.

Aufgrund der Fähigkeit eine Sorbitol-Akkumulation zu bewirken, eignen sich die erfindungsgemäßen Verbindungen insbesonderer als Tool im pharmakologischen Modell zur Prüfung von Aldose-Reductase-Inhibitoren. Die Erfindung betrifft daher auch diese Verwendung der Pyrimidin-Derivate der Formel I und von deren pharmakologisch verträglichen Salzen.

Außerdem in den Beispielen aufgeführten Verbindungen können die in der folgenden Tabelle zusammengestellten Verbindungen der allgemeinen Formel I bzw. deren Salze erhalten werden.

Verwendete Abkürzungen: Methyl (Me), Ethyl (Et), Propyl (Pr), Butyl (Bu), Hexyl (Hex), Acetyl (Ac), Phenyl (Ph), iso (i) und cyclo (c).

Tabelle

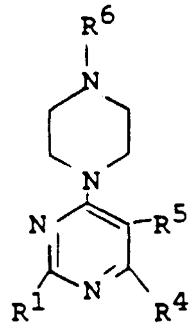

(Formel I mit $R^2R^3$ =

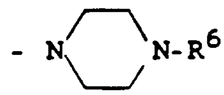 )

| $R^1$ | $R^4$ | $R^5$ | $R^6$ |
|---|---|---|---|
| H | H | H | H |
| H | H | H | 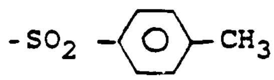 |
| H | H | H | 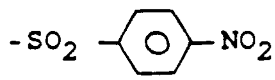 |
| H | H | H | $-SO_2 - CH_3$ |
| H | H | H | $-SO_2 - N(Me)_2$ |
| H | H | H | $-SO_2 - NHCH_3$ |
| H | H | H | 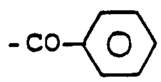 |
| H | H | H | $-CO-CH_3$ |
| H | H | H | 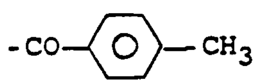 |
| H | H | H | 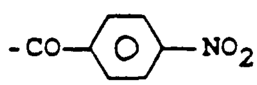 |
| H | H | H | 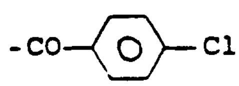 |
| H | H | H | $-SO_2 - N(Et)_2$ |

Fortsetzung der Tabelle

| $R^1$ | $R^4$ | $R^5$ | $R^6$ |
|---|---|---|---|
| H | H | H | $-SO_2-N(iPr)_2$ |
| H | H | H | 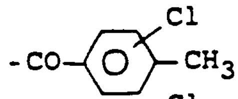 |
| H | H | H | 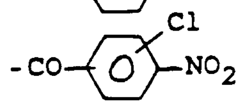 |
| H | H | H | -CO - NHEt |
| $CH_3$ | H | H | H |
| $CH_3$ | H | H | $-SO_2-NHCH_3$ |
| $CH_3$ | H | H | 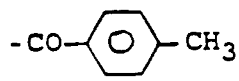 |
| $CH_3$ | H | H | 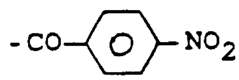 |
| $CH_3$ | H | H | 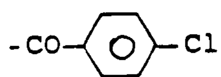 |
| $CH_3$ | H | H | 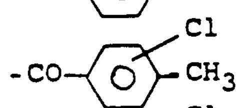 |
| $CH_3$ | H | H | 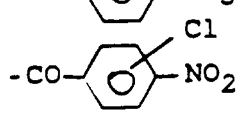 |
| $CH_3$ | H | H | $-SO_2-N(Et)_2$ |
| $CH_3$ | H | H | $-CO-CH_3$ |
| $CH_3$ | H | H | $-CO-N(Me)_2$ |
| $CH_3$ | H | H | 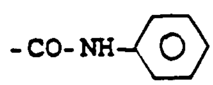 |
| $CH_3$ | H | H | 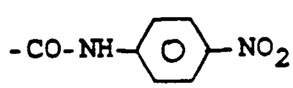 |

Fortsetzung der Tabelle

| $R^1$ | $R^4$ | $R^5$ | $R^6$ |
|---|---|---|---|
| $CH_3$ | H | H | 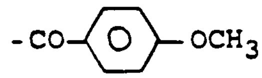 |
| $CH_3$ | H | H | 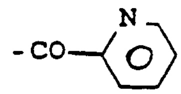 |
| $CH_3$ | H | H | 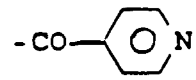 |
| Et | H | H | H |
| Et | H | H | 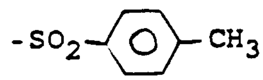 |
| Et | H | H | 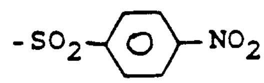 |
| Et | H | H | $-SO_2-CH_3$ |
| Et | H | H | $-SO_2-NH(CH_3$ |
| Et | H | H | $-SO_2-N(CH_3)_2$ |
| Et | H | H | $-SO_2-NEt_2$ |
| Et | H | H | $-SO_2-N(iPr)_2$ |
| Et | H | H | $-SO_2-Et$ |
| Et | H | H | $-COCH_3$ |
| Et | H | H | 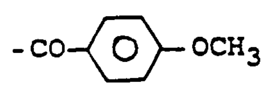 |
| Et | H | H | 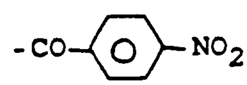 |
| Et | H | H | 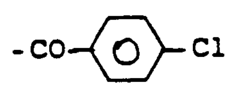 |

Fortsetzung der Tabelle

| $R^1$ | $R^4$ | $R^5$ | $R^6$ |
|---|---|---|---|
| Et | H | H | 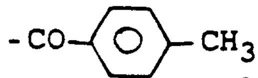 |
| Et | H | H | 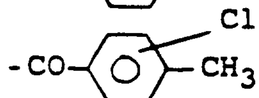 |
| Et | H | H | 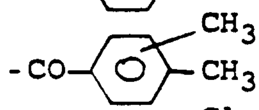 |
| Et | H | H | 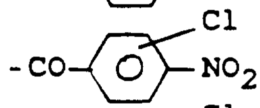 |
| Et | H | H | 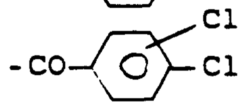 |
| Et | H | H | 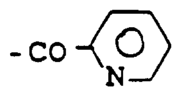 |
| Et | H | H | 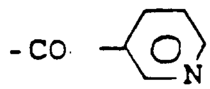 |
| Et | H | H | 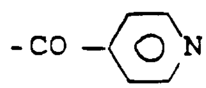 |

Die nachstehenden Beispiele dienen zur Eläuterung der Erfindung.

Beispiel 1

2-Methyl-4-(4-N,N-dimethylsulfamoyl-piperazino)-pyrimidin und das entsprechende Hydrochlorid

a) 4-Hydroxy-2-methyl-pyrimidin

Eine Suspension aus 240 g Natriumhydrid (55 %ige Suspension) in 5 l Toluol wurde bei Raumtemperatur unter Rühren mit einer Mischung aus 555 g Ameisensäureethylester und 440 g Essigsäureethylester tropfenweise versetzt, bis die Wasserstoffentwicklung beendet war. Es wurde 1 h nachgerührt, der Niederschlag abgesaugt und mit Ether gewaschen. Es wurden 650 g Formylessigsäureethylester-Natriumsalz erhalten, das in 4 l Wasser gelöst und mit 475 g Acetamidin-Hydrochlorid umgesetzt wurde. Die Reaktionslösung wurde 2 Tage lang bei Raumtemperatur stehen gelassen, dann wurde das Wasser im Vakuum abdestilliert, und der Rückstand über Kieselgel chromatographiert.
Es wurden 240 g 4-Hydroxy-2-methyl-pyrimidin erhalten.
(Schmp.: 214 °C)

b) 4-Chlor-2-methyl-pyrimidin

11 g 4-Hydroxy-2-methyl-pyrimidin wurden mit 50 ml Phosphoroxychlorid versetzt und langsam auf 80 °C erhitzt. Nachdem sich der Feststoff vollständig gelöst hatte, wurde überschüssiges Phosphoroxychlorid im Vakuum abdestilliert und der Rückstand wurde auf Eis gegeben. Die wässrige Phase wurde mehrmals mit Dichlormethan extrahiert, die organischen Phasen wurden über Natriumsulfat getrocknet, filtriert und eingeengt.
Es wurden 8 g 4-Chlor-2-methyl-pyrimidin erhalten.
(Schmp.: 59 °C)

c) 2-Methyl-4-piperazino-pyrimidin

13 g 4-Chlor-2-methyl-pyrimidin wurden in 200 ml Tetrahydrofuran gelöst und mit 17,5 g Piperazin versetzt. Das Reaktionsgemisch wurde 24 h unter Rückfluß erhitzt. Das ausgefallene Piperazin-Hydrochlorid wurde abgesaugt und mit Tetrahydrofuran gewaschen. Nach dem Einengen der Lösung im Vakuum wurden 19 g 2-Methyl-4-piperazino-pyrimidin erhalten, das ohne weitere Reinigung umgesetzt wurde.

d) 2-Methyl-4-(4-N,N-dimethylsulfamoyl-piperazino)-pyrimidin

5 g 2-Methyl-4-piperazino-pyrimidin wurden in 80 ml Pyridin gelöst und bei Raumtemperatur mit 4,7 g N,N-Dimethylamidosulfonsäurechlorid versetzt. Die Reaktionslösung wurde 5 h auf 50 °C erhitzt. Nachdem sich die Ausgangsverbindung vollständig umgesetzt hatte, wurde das Reaktionsgemisch nach dem Abkühlen auf Raumtemperatur mit Diethylether versetzt. Die ausgefallenen Kristalle wurden abgesaugt. Nach säulechromatographischer Reinigung wurden 2,6 g 2-Methyl-4-(4-N,N-dimethylsulfamoyl-piperazino)-pyrimidin erhalten.
(Schmp.: 114 °C)

e) 2-Methyl-4-(N,N-dimethylsulfamoyl-piperazino)-pyrimidin-Hydrochlorid

1 g 2-Methyl-4-(N,N-dimethylsulfamoyl-piperazino)-pyrimidin wurden in 5 ml Methanol gelöst und bei Raumtemperatur unter Rühren mit 10 ml methanolischer Salzsäure versetzt. Nach 15 Minuten wurde das Lösungsmittel im Vakuum abdestilliert und der Rückstand mit Aceton versetzt. Es wurden 1 g Hydrochlorid als weiße Kristalle isoliert.
(Schmp.: 238 °C, Zers.)

Beispiel 2

2-Methyl-4-(4-benzoyl-piperazino)-pyrimidin
1 g 2-Methyl-4-piperazino-pyrimidin wurden in 50 ml Aceton gelöst und mit 2 g Kaliumcarbonat und 0,8 g Benzoylchlorid versetzt. Die Suspension wurde 6 h unter Rückfluß erhitzt, bis keine Ausgangsverbindung mehr nachweisbar war. Nach der Filtration wurde das Filtrat im Vakuum eingeengt und der Rückstand aus Dichlormethan/Petrolether umkristallisiert. Es wurden 0,5 g 2-Methyl-4-(4-benzoyl-piperazino)-pyrimidin erhalten.
(Schmp.: 147 °C)
Auf anloge Weise wurden die folgenden Verbindungen hergestellt.

Beispiel 3

2-Methyl-4-(4-ethylcarbamoyl-piperazino)-pyrimidin (Schmp.: 138 °C)

Beispiel 4

2-Methyl-4-(4-methansulfonylpiperazino)-pyrimidin (Schmp.: 241 °C) (Zers.)

Beispiel 5

2-Methyl-4-[4-(4-nitrobenzolsulfonyl)-piperazino]-pyrimidin (Schmp.: 166 °C)

Beispiel 6

2-Methyl-4-[4-(p-toluolsulfonyl)-piperazino]-pyrimidin (Schmp.: 142 °C)

Beispiel 7

2-Methyl-4-(4-nicotinoyl-piperazino)-pyrimidin (Schmp.: 118 °C)

**Patentansprüche**

**Patentansprüche für folgende Vertragsstaaten : AT, BE, CH, DE, DK, FR, GB, GR, IT, LI, LU, NL, SE**

**1.** Pyrimidin-Derivate der Formel I

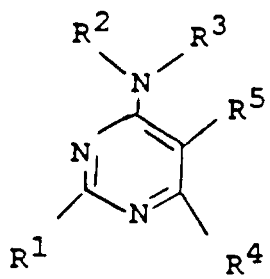

I

in welcher

$R^1$ und $R^5$ gleich sind oder verschieden sind und Wasserstoff oder $(C_1-C_6)$-Alkyl bedeuten, $R^4$ Wasserstoff ist,

$R^2$, $R^3$ zusammen mit dem Stickstoff, an den sie gebunden sind, die Piperazinogruppe bilden, oder eine mit gleichen oder verschiedenen Gruppen $R^6$ und $R^7$ substituierte Piperazinogruppe darstellen, wobei $R^6$, $R^7$ $(C_1-c_6)$-Alkyl, Sulfamoyl, N-$(C_1-C_4)$-Alkylsulfamoyl, N,N-$(C_1-C_4)$-Dialkylsulfamoyl, $(C_1-C_6)$-Alkoxycarbonyl, N,N-$(C_1-C_4)$-Dialkylcarbamoyl, N-$(C_1-C_4)$-Alkylcarbamoyl, N-$(C_6-C_{12})$-Arylcarbamoyl, im Arylrest mit $(C_1-C_4)$-Alkyl, $(C_1-C_4)$-Alkoxy, Halogen, $NO_2$, $NH_2$, CN oder $CF_3$ substituiertes $(C_6-C_{12})$-Arylcarbamoyl, Carbamoyl, $(C_1-C_6)$-Alkylcarbonyl, $(C_6-C_{12})$-Arylcarbonyl, im Arylrest mit $(C_1-C_4)$-Alkyl, $(C_1-C_4)$-Alkoxy, Halogen, $NO_2$, $NH_2$, CN oder $CF_3$ substituiertes $(C_6-C_{12})$-Arylcarbonyl, $(C_1-C_6)$-Alkylsulfonyl, $(C_1-C_6)$-Alkylsulfinyl, $(C_6-C_{12})$-Arylsulfonyl, im Arylrest mit $(C_1-C_4)$-Alkyl, $(C_1-C_4)$-Alkoxy, Halogen, $NO_2$, $NH_2$, CN oder $CF_3$ substituiertes $(C_6-C_{12})$-Arylsulfonyl, unsubstituiertes Heteroarylcarbonyl oder Heteroarylsulfonyl, wobei die Heteroarylreste ein Sauerstoffatom oder 1 bis 3 Stickstoffatome aufweisen, bedeuten oder einer der Substituenten $R^6$, $R^7$ Wasserstoff ist, sowie deren physiologisch verträgliche Salze.

**2.** Pyrimidin-Derivate gemäß Anspruch 1 und deren physiologisch verträgliche Salze, dadurch gekennzeichnet, daß in Formel I die Substituenten folgende Bedeutungen haben:

$R^1$ und $R^5$ (gleich oder verschieden), Wasserstoff oder $(C_1-C_6)$-Alkyl

$R^4$ Wasserstoff und

$R^2$, $R^3$ zusammen mit dem Stickstoff, an den sie gebundenen sind, einen Piperazin-Ring, der gegebenenfalls durch gleich oder verschiedene Gruppen $R_6$ und $R^7$ substituiert ist, wobei

$R^6$, $R^7$ $(C_1-C_6)$-Alkyl, Sufamoyl, N-$(C_1-C_4)$-Alkylsulfamoyl, N,N-$(C_1-C_4)$-Dialkylsulfamoyl, $(C_1-C_6)$-Alkoxycarbonyl, N,N-$(C_1-C_4)$-Dialkylcarbamoyl, N-$(C_1-C_4)$-Alkylcarbamoyl, Carbamoyl, $(C_1-C_6)$-Alkylcarbonyl, $(C_6-C_{12})$-Arylcarbonyl, im Arylrest mit $(C_1-C_4)$-Alkyl, $(C_1-C_4)$-Alkoxy, Halogen, $NO_2$, $NH_2$, CN oder $CF_3$ substituiertes $(C_6-C_{12})$Arylcarbonyl, $(C_1-C_6)$-Alkylsulfonyl, $(C_1-C_6)$-Alkylsulfinyl, $(C_6-C_{12})$-Arylsulfonyl, im Arylrest mit $(C_1-C_4)$-Alkyl, $(C_1-C_4)$Alkoxy, Halogen, $NO_2$, $NH_2$, CN oder $CF_3$ substituiertes $(C_6-C_{12})$-Arylsulfonyl, unsubstituiertes Heteroarylcarbonyl oder Heteroarylsulfonyl, wobei die Heteroarylreste ein Sauerstoffatom oder 1 bis 3 Stickstoffatome aufweisen, bedeuten oder einer der Substituenten $R^6$, $R^7$ Wasserstoff ist.

**3.** Pyrimidin-Derivate gemäß Anspruch 1 und deren physiologisch verträgliche Salze, dadurch gekennzeichnet, daß in Formel I die Substituenten folgende Bedeutungen haben:

$R^1$ und $R^5$ (gleich oder verschieden), Wasserstoff oder $(C_1-C_4)$-Alkyl,

$R^4$ Wasserstoff und

$R^2$, $R^3$ zusammen mit dem Stickstoff an den sie gebunden sind einen Piperazin-Ring, der gegebenenfalls in Position 4 einen weiteren Substituenten $R^6$ trägt, wobei

$R^6$ Sulfamoyl, N-$(C_1-C_4)$-Alkylsulfamoyl, N,N-$(C_1-C_4)$-Dialkylsulfamoyl, Carbamoyl, N-$(C_1-$

$C_4$)-Alkylcarbamoyl, N,N($C_1$-$C_4$)-Dialkylcarbamoyl, ($C_1$-$C_6$)-Alkylcarbonyl, ($C_6$-$C_{12}$)-Arylcarbonyl, im Arylrest mit ($C_1$-$C_4$)-Alkyl, ($C_1$-$C_4$)-Alkoxy, Halogen, $NO_2$, $NH_2$, CN oder $CF_3$ substituiertes ($C_6$-$C_{12}$)-Arylcarbonyl oder Pyridincarbonyl bedeutet.

**4.** Pyrimidin-Derivate gemäß Anspruch 1 und deren physiologisch verträgliche Salze, dadurch gekennzeichnet, daß in Formel I die Substituenten folgende Bedeutungen haben:

$R^1$ Wasserstoff oder ($C_1$-$C_2$)-Alkyl,

$R^4$ Wasserstoff

$R^5$ Wasserstoff,

$R^2$, $R^3$ zusammen mit dem Stickstoff, an den sie gebunden sind, einen Piperazin-Ring bilden, der gegebenenfalls in Position 4 einen weiteren Substituenten $R^6$ trägt, wobei $R^6$ N-($C_1$-$C_3$)-Alkylsulfamoyl, N,N-($C_1$-$C_2$)-Dialkylsulfamoyl, N-($C_1$-$C_2$)-Alkylcarbamoyl, N,N-($C_1$-$C_2$)-Dialkylcarbamoyl, ($C_1$-$C_2$)-Alkylcarbonyl, Phenylcarbonyl, das gegebenenfalls im Phenylrest substituiert ist mit ($C_1$-$C_2$)-Alkyl, Chlor oder $NO_2$, oder Pyridincarbonyl ist, insbesondere N,N-Dimethylsulfamoyl, Phenylcarbonyl oder Pyridincarbonyl.

**5.** Pyrimidin-Derivate gemäß Anspruch 4 und deren physiologisch verträgliche Salze, dadurch gekennzeichnet, daß in Formel I $R^1$ Methyl bedeutet.

**6.** Verfahren zur Herstellung von Verbindungen der Formel I gemäß Anspruch 1, dadurch gekennzeichnet, daß man in an sich bekannter Weise

a) eine Verbindung der Formel II

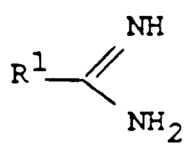

II

worin $R^1$ die zu Formel I angegebenen Bedeutungen hat oder deren Säureadditionssalz, mit einer Verbindung der Formel III

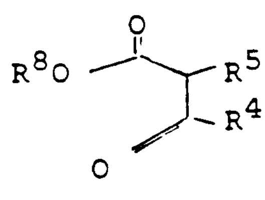

III

worin $R^4$ und $R^5$ die zu Formel I angegebenen Bedeutungen haben und $R^8$ Methyl oder Ethyl bedeutet, oder mit deren Basensalz zu einer Verbindung der Formel IV

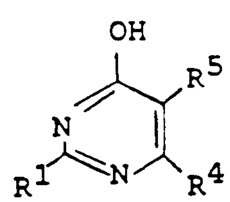

IV

worin $R^1$, $R^4$ und $R^5$ die zur Formel I angegebenen Bedeutungen hat, umsetzt

b) eine erhaltene Verbindung IV mit einem anorganischen Säurechlorid zu einem Pyrimidin-Derivat der Formel V

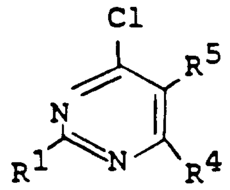

V

in welcher die Reste $R^1$, $R^4$ und $R^5$ die zu Formel I angegebenen Bedeutungen haben, umsetzt,
c) eine erhaltene Verbindung der Formel V mit einem Amin der Formel VI

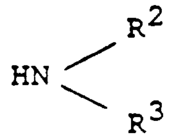

VI

in der $R^2$ und $R^3$ die zu Formel I angegebenen Bedeutungen hat, zu einer Verbindung der Formel I umsetzt, und
d) gegebenenfalls in einer erhaltenen Verbindung der Formel I, worin $R^2$ und $R^3$ gemeinsam mit dem sie tragenden Stickstoffatom den Piperazinorest bilden, den oder die Reste $R^6$/$R^7$ einführt, und
e) gegebenenfalls eine erhaltene Verbindung der Formel I in ein physiologisch verträgliches Salz überführt.

**7.** Verwendung von Verbindungen der Formel I gemäß Anspruch 1 und von deren Salzen als Tool im pharmakologischen Modell.

**Patentansprüche für folgenden Vertragsstaat : ES**

**1.** Verfahren zur Herstellung von Pyrimidin-Derivaten der Formel I

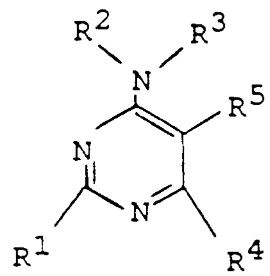

I

in welcher

$R^1$ und $R^5$ gleich sind oder verschieden sind und Wasserstoff oder $(C_1$-$C_6)$-Alkyl bedeuten, $R^4$ Wasserstoff ist,

$R^2$, $R^3$ zusammen mit dem Stickstoff, an den sie gebunden sind, die Piperazinogruppe bilden oder eine mit gleicher oder verschiedenen Gruppen $R^6$ und $R^7$ substituierte Piperazinogruppe darstellen, wobei $R^6$, $R^7$ $(C_1$-$C_6)$-Alkyl, Sulfamoyl, N-$(C_1$-$C_4)$-Alkylsulfamoyl, N,N-$(C_1$-$C_4)$-Dialkylsulfamoyl, $(C_1$-$C_6)$-Alkoxycarbonyl, N,N-$(C_1$-$C_4)$-Dialkylcarbamoyl, N-$(C_1$-$C_4)$-Alkylcarbamoyl, N-$(C_6$-$C_{12})$-Arylcarbamoyl, im Arylrest mit $(C_1$-$C_4)$-Alkyl, $(C_1$-$C_4)$-Alkoxy, Halogen, $NO_2$, $NH_2$, CN oder $CF_3$ substituiertes $(C_6$-$C_{12})$-Arylcarbamoyl, Carbamoyl, $(C_1$-$C_6)$-Alkylcarbonyl, $(C_6$-$C_{12})$-Arylcarbonyl, im Arylrest mit $(C_1$-$C_4)$-Alkyl, $(C_1$-$C_4)$-Alkoxy, Halogen, $NO_2$, $NH_2$, CN oder $CF_3$ substituiertes $(C_6$-$C_{12})$-Arylcarbonyl, $(C_1$-$C_6)$-Alkylsulfonyl, $(C_1$-$C_6)$-Alkylsulfinyl, $(C_6$-$C_{12})$-Arylsulfonyl, im Arylrest mit $(C_1$-$C_4)$-Alkyl, $(C_1$-$C_4)$-Alkoxy, Halogen, $NO_2$, $NH_2$, CN oder $CF_3$ substituiertes $(C_6$-$C_{12})$-Arylsulfonyl, unsubstituiertes Heteroarylcarbonyl oder Heteroarylsulfonyl, wobei die Heteroarylreste ein Sauerstoffatom oder 1 bis 3 Stickstoffatome

aufweisen, bedeuten oder einer der Substituenten $R^6$, $R^7$ Wasserstoff ist, sowie von deren physiologisch verträglichen Salzen, dadurch gekennzeichnet, daß man

a) eine Verbindung der Formel II

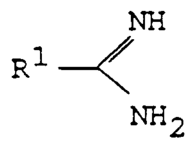

II

worin $R^1$ die zu Formel I angegebenen Bedeutungen hat oder deren Säureadditionssalz, mit einer Verbindung der Formel III

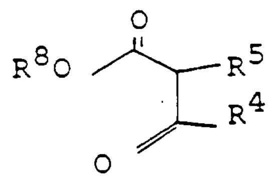

III

worin $R^4$ und $R^5$ die zu Formel I angegebenen Bedeutungen haben und $R^8$ Methyl oder Ethyl bedeutet, oder mit deren Basensalz zu einer Verbindung der Formel IV

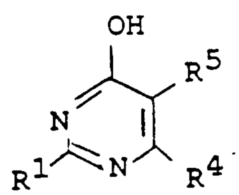

IV

worin $R^1$, $R^4$ und $R^5$ die zur Formel I angegebenen Bedeutungen hat, umsetzt

b) eine erhaltene Verbindung IV mit einem anorganischen Säurechlorid zu einem Pyrimidin-Derivat der Formel V

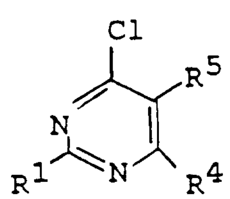

V

in welcher die Reste $R^1$, $R^4$ und $R^5$ die zu Formel I angegebenen Bedeutungen haben, umsetzt,

c) eine erhaltene Verbindung der Formel V mit einem Amin der Formel VI

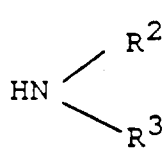

VI

in der $R^2$ und $R^3$ die zu Formel I angegebenen Bedeutungen hat, zu einer Verbindung der Formel I umsetzt, und

d) gegebenenfalls in einer erhaltenen Verbindung der Formel I, worin $R^2$ und $R^3$ gemeinsam mit dem sie tragenden Stickstoffatom den Piperazinorest bilden, den oder die Reste $R^6/R^7$ einführt, und

e) gegebenenfalls eine erhaltene Verbindung der Formel I in ein physiologisch verträgliches Salz überführt.

**2.** Verfahren gemäß Anspruch 1, dadurch gekennzeichnet, daß man eine Verbindung in Formel I, wobei in Formel I die Substituenten folgende Bedeutungen haben:

$R^1$ und $R^5$ (gleich oder verschieden), Wasserstoff oder $(C_1-C_6)$-Alkyl, und

$R^4$ Wasserstoff und

$R^2$, $R^3$ zusammen mit dem Stickstoff, an den sie gebundenen sind, einen Piperazin-Ring, der gegebenenfalls durch gleich oder verschiedene Gruppen $R_6$ und $R^7$ substituiert ist, wobei

$R^6$, $R^7$ $(C_1-C_6)$-Alkyl, Sufamoyl, N-$(C_1-C_4)$-Alkylsulfamoyl, N,N-$(C_1-C_4)$-Dialkylsulfamoyl, $(C_1-C_6)$-Alkoxycarbonyl, N,N-$(C_1-C_4)$-Dialkylcarbamoyl, N-$(C_1-C_4)$-Alkylcarbamoyl, Carbamoyl, $(C_1-C_6)$-Alkylcarbonyl, $(C_6-C_{12})$-Arylcarbonyl, im Arylrest mit $(C_1-C_4)$-Alkyl, $(C_1-C_4)$-Alkoxy, Halogen, $NO_2$, $NH_2$, CN oder $CF_3$ substituiertes $(C_6-C_{12})$Arylcarbonyl, $(C_1-C_6)$-Alkylsulfonyl, $(C_1-C_6)$-Alkylsulfinyl, $(C_6-C_{12})$-Arylsulfonyl, im Arylrest mit $(C_1-C_4)$-Alkyl, $(C_1-C_4)$Alkoxy, Halogen, $NO_2$, $NH_2$, CN oder $CF_3$ substituiertes $(C_6-C_{12})$-Arylsulfonyl, unsubstituiertes Heteroarylcarbonyl oder Heteroarylsulfonyl, wobei die Heteroarylreste ein Sauerstoffatom oder 1 bis 3 Stickstoffatome aufweisen, bedeuten oder einer der Substituenten $R^6$, $R^7$ Wasserstoff ist, oder deren physiologisch verträgliche Salze herstellt.

**3.** Verfahren gemäß Anspruch 1 , dadurch gekennzeichnet, daß man eine Verbindung der Formel I, wobei die Substituenten folgende Bedeutungen haben

$R^1$ und $R^5$ (gleich oder verschieden), Wasserstoff oder $(C_1-C_4)$-Alkyl,

$R^4$ Wasserstoff und

$R^2$, $R^3$ zusammen mit dem Stickstoff an den sie gebunden sind einen Piperazin-Ring, der gegebenenfalls in Position 4 einen weiteren Substituenten $R^6$ trägt, wobei

$R^6$ Sulfamoyl, N-$(C_1-C_4)$-Alkylsulfamoyl, N,N-$(C_1-C_4)$-Dialkylsulfamoyl, Carbamoyl, N-$(C_1-C_4)$-Alkylcarbamoyl, N,N$(C_1-C_4)$-Dialkylcarbamoyl, $(C_1-C_6)$-Alkylcarbonyl, $(C_6-C_{12})$-Arylcarbonyl, im Arylrest mit $(C_1-C_4)$-Alkyl, $(C_1-C_4)$-Alkoxy, Halogen, $NO_2$, $NH_2$, CN oder $CF_3$ substituiertes $(C_6-C_{12})$-Arylcarbonyl oder Pyridincarbonyl bedeutet, oder deren physilogisch verträgliche Salze herstellt.

**4.** Verfahren gemäß Anspruch 1, dadurch gekennzeichnet, daß man eine Verbindung der Formel I, wobei in Formel I die Substituenten folgende Bedeutungen haben:

$R^1$ Wasserstoff oder $(C_1-C_2)$-Alkyl

$R^4$ Wasserstoff,

$R^5$ Wasserstoff,

$R^2$, $R^3$ zusammen mit dem Stickstoff, an den sie gebunden sind, einen Piperazin-Ring bilden, der gegebenenfalls in Position 4 einen weiteren Substitueten $R^6$ trägt, wobei $R^6$ N-$(C_1-C_3)$-Alkylsulfamoyl, N,N-$(C_1-C_2)$-Dialkylsulfamoyl, N-$(C_1-C_2)$-Alkylcarbamoyl, N,N-$(C_1-C_2)$-Dialkylcarbamoyl, $(C_1-C_2)$-Alkylcarbonyl, Phenylcarbonyl, das gegebenenfalls im Phenylrest substituiert ist mit $(C_1-C_2)$-Alkyl, Chlor oder $NO_2$, oder Pyridincarbonyl ist, insbesondere N,N-Dimethylsulfamoyl, Phenylcarbonyl oder Pyridincarbonyl, oder deren physiologisch verträgliche Salze herstellt.

**5.** Verfahren gemäß Anspruch 4, dadurch gekennzeichnet, daß man eine Verbindung der Formel I mit $R^1$ Methyl, oder deren physiologisch verträglichen Salze herstellt.

**6.** Verwendung von Verbindungen der Formel I gemäß Anspruch 1 und von deren Salzen als Tool im pharmakologischen Modell.

## Claims

**Claims for the following Contracting States : AT, BE, CH, DE, DK, FR, GB, GR, IT, LI, LU, NL, SE**

**1.** A pyrimidine derivative of the formula I

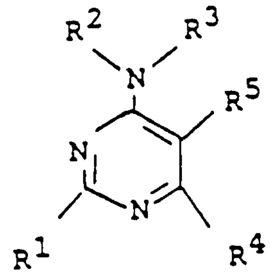

I

in which

| | |
|---|---|
| $R^1$ and $R^5$ | are identical or different and denote hydrogen or $(C_1\text{-}C_6)$-alkyl, $R^4$ is hydrogen, |
| $R^2$, $R^3$ | form, together with the nitrogen to which they are bonded, the piperazino group, or a piperazino group which is substituted by identical or different groups $R^6$ and $R^7$, where $R^6$, $R^7$ denote $(C_1\text{-}C_6)$-alkyl, sulfamoyl, N-$(C_1\text{-}C_4)$-alkylsulfamoyl, N,N-$(C_1\text{-}C_4)$-dialkylsulfamoyl, $(C_1\text{-}C_6)$-alkoxycarbonyl, N,N-$(C_1\text{-}C_4)$-dialkylcarbamoyl, N-$(C_1\text{-}C_4)$-alkylcarbamoyl, N-$(C_6\text{-}C_{12})$-arylcarbamoyl, or $(C_6\text{-}C_{12})$-arylcarbamoyl which is substituted in the aryl radical by $(C_1\text{-}C_4)$-alkyl, $(C_1\text{-}C_4)$-alkoxy, halogen, $NO_2$, $NH_2$, CN or $CF_3$, or carbamoyl, $(C_1\text{-}C_6)$-alkylcarbonyl, or $(C_6\text{-}C_{12})$-arylcarbonyl, $(C_6\text{-}C_{12})$-arylcarbonyl which is substituted in the aryl radical by $(C_1\text{-}C_4)$-alkyl, $(C_1\text{-}C_4)$-alkoxy, halogen, $NO_2$, $NH_2$, CN or $CF_3$, or $(C_1\text{-}C_6)$-alkylsul-fonyl, $(C_1\text{-}C_6)$-alkylsulfinyl, $(C_6\text{-}C_{12})$-arylsulfonyl, or $(C_6\text{-}C_{12})$-arylsulfonyl which is substituted in the aryl radical by $(C_1\text{-}C_4)$-alkyl, $(C_1\text{-}C_4)$-alkoxy, halogen, $NO_2$, $NH_2$, CN or $CF_3$, or unsubstituted heteroarylcarbonyl or heteroarylsulfonyl in which the heteroaryl radicals contain one oxygen atom or 1 to 3 nitrogen atoms, or one of the substituents $R^6$, $R^7$ is hydrogen, as well as the physiologically tolerated salts thereof. |

**2.** A pyrimidine derivative as claimed in claim 1, and the physiologically tolerated salts thereof, wherein in formula I the substituents have the following meanings:

| | |
|---|---|
| $R^1$ and $R^5$ | (identical or different), hydrogen or $(C_1\text{-}C_6)$-alkyl, |
| $R^4$ | hydrogen and |
| $R^2$, $R^3$ | together with the nitrogen to which they are bonded, a piperazine ring which is optionally substituted by identical or different groups $R^6$ and $R^7$, where |
| $R^6$, $R^7$ | denote $(C_1\text{-}C_6)$-alkyl, sulfamoyl, N-$(C_1\text{-}C_4)$-alkylsulfamoyl, N,N-$(C_1\text{-}C_4)$-dialkylsulfamoyl, $(C_1\text{-}C_6)$-alkoxycarbonyl, N,N-$(C_1\text{-}C_4)$-dialkylcarbamoyl, N-$(C_1\text{-}C_4)$-alkylcarbamoyl, carbamoyl, $(C_1\text{-}C_6)$-alkylcarbonyl, $(C_6\text{-}C_{12})$-arylcarbonyl, $(C_6\text{-}C_{12})$-arylcarbonyl which is substituted in the aryl radical by $(C_1\text{-}C_4)$-alkyl, $(C_1\text{-}C_4)$-alkoxy, halogen, $NO_2$, $NH_2$,CN or $CF_3$, or $(C_1\text{-}C_6)$ - alkylsulfonyl, $(C_1\text{-}C_6)$-alkylsulfinyl, $(C_6\text{-}C_{12})$-arylsulfonyl, or $(C_6\text{-}C_{12})$-arylsulfonyl which is substituted in the aryl radical by $(C_1\text{-}C_4)$-alkyl, $(C_1\text{-}C_4)$-alkoxy, halogen, $NO_2$, $NH_2$, CN or $CF_3$, or unsubstituted heteroarylcarbonyl or heteroarylsulfonyl in which the heteroaryl radicals contain one oxygen atom or 1 to 3 nitrogen atoms, or one of the substituents $R^6$, $R^7$ is hydrogen. |

**3.** A pyrimidine derivative as claimed in claim 1, and the physiologically tolerated salts thereof, wherein in formula I the substituents have the following meanings:

| | |
|---|---|
| $R^1$ and $R^5$ | (identical or different), hydrogen or $(C_1\text{-}C_4)$-alkyl, |
| $R^4$ | hydrogen and |
| $R^2$, $R^3$ | together with the nitrogen to which they are bonded, a piperazine ring which optionally carries in position 4 another substituent $R^6$, where |
| $R^6$ | denotes sulfamoyl, N-$(C_1\text{-}C_4)$-alkylsulfamoyl, N,N-$(C_1\text{-}C_4)$-dialkylsulfamoyl, carbamoyl, N-$(C_1\text{-}C_4)$-alkylcarbamoyl, N,N-$(C_1\text{-}C_4)$-dialkylcarbamoyl, $(C_1\text{-}C_6)$-alkylcarbonyl, $(C_6\text{-}C_{12})$-arylcarbonyl, or $(C_6\text{-}C_{12})$-arylcarbonyl which is substituted in the aryl radical by $(C_1\text{-}C_4)$-alkyl, $(C_1\text{-}C_4)$-alkoxy, halogen, $NO_2$, $NH_2$, CN or $CF_3$, or pyridinecarbonyl. |

**4.** A pyrimidine derivative as claimed in claim 1, and the physiologically tolerated salts thereof, wherein in formula I the substituents have the following meanings:

$R^1$ hydrogen or ($C_1$-$C_2$)-alkyl,

$R^4$ hydrogen,

$R^5$ hydrogen,

$R^2$, $R^3$ form, together with the nitrogen to which they are bonded, a piperazine ring which optionally carries in position 4 another substituent $R^6$, where $R^6$ represents N-($C_1$-$C_3$)-alkylsulfamoyl, N,N($C_1$-$C_2$)-dialkylsulfamoyl, N-($C_1$-$C_2$)-alkylcarbamoyl, N,N-($C_1$-$C_2$)-dialkylcarbamoyl, ($C_1$-$C_2$)alkylcarbonyl, phenylcarbonyl which is optionally substituted in the phenyl radical by ($C_1$-$C_2$)-alkyl, chlorine or $NO_2$, or pyridinecarbonyl, in particular N,N-dimethylsulfamoyl, phenylcarbonyl or pyridinecarbonyl.

**5.** A pyrimidine derivative as claimed in claim 4, and the physiologically tolerated salts thereof, wherein in formula I $R^1$ is methyl.

**6.** A process for the preparation of compounds of the formula I as claimed in claim 1, which comprises, in a manner known per se,

a) reacting a compound of the formula II

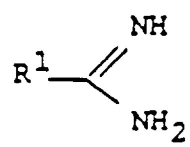

II

in which $R^1$ has the meanings indicated for formula I, or the acid addition salt thereof, with a compound of the formula III

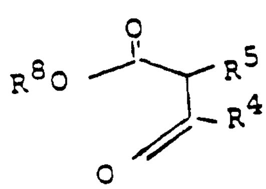

III

in which $R^4$ and $R^5$ have the meanings indicated for formula I, and $R^8$ denotes methyl or ethyl, or with a base salt thereof, to give a compound of the formula IV

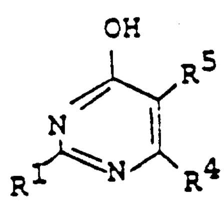

IV

in which $R^1$, $R^4$ and $R^5$ have the meanings indicated for formula I,

b) reacting a resulting compound IV with an inorganic acid chloride to give a pyrimidine derivative of the formula V

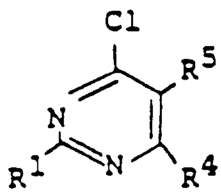

V

in which the radicals $R^1$, $R^4$ and $R^5$ have the meanings indicated for formula I,
c) reacting a resulting compound of the formula V with an amine of the formula VI

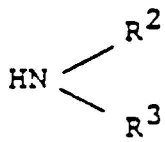

VI

in which $R^2$ and $R^3$ have the meanings indicated for formula I, to give a compound of the formula I, and
d) where appropriate introducing the radical(s) $R^6$/$R^7$ into a resulting compound of the formula I in which $R^2$ and $R^3$ form, together with the nitrogen atom carrying them, the piperazino radical, and
e) where appropriate converting a resulting compound of the formula I into a physiologically tolerated salt.

**7.** The use of compounds of the formula I as claimed in claim 1 and of the salts thereof as a tool in a pharmacological model.

**Claims for the following Contracting State : ES**

**1.** A process for the preparation of a pyrimidine derivative of the formula I

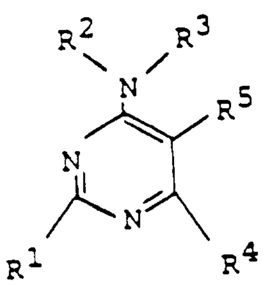

I

in which

$R^1$ and $R^5$ are identical or different and denote hydrogen or $(C_1\text{-}C_6)$-alkyl, $R^4$ is hydrogen,

$R^2$, $R^3$ form, together with the nitrogen to which they are bonded, the piperazino group, or piperazino group which is substituted by identical or different groups $R^6$ and $R^7$, where $R^6$, $R^7$ denote $(C_1\text{-}C_6)$-alkyl, sulfamoyl, N-$(C_1\text{-}C_4)$-alkylsulfamoyl, N,N-$(C_1\text{-}C_4)$-dialkylsulfamoyl, $(C_1\text{-}C_6)$-alkoxycarbonyl, N,N-$(C_1\text{-}C_4)$-dialkylcarbamoyl, N-$(C_1\text{-}C_4)$-alkylcarbamoyl, N-$(C_6\text{-}C_{12})$-arylcarbamoyl, or $(C_6\text{-}C_{12})$-arylcarbamoyl which is substituted in the aryl radical by $(C_1\text{-}C_4)$-alkyl, $(C_1\text{-}C_4)$-alkoxy, halogen, $NO_2$, $NH_2$, CN or $CF_3$, or carbamoyl, $(C_1\text{-}C_6)$-alkylcarbonyl, or $(C_6\text{-}C_{12})$-arylcarbonyl, $(C_6\text{-}C_{12})$-arylcarbonyl which is substituted in the aryl radical by $(C_1\text{-}C_4)$-alkyl, $(C_1\text{-}C_4)$-alkoxy, halogen, $NO_2$, $NH_2$, CN or $CF_3$, or $(C_1\text{-}C_6)$-alkylsul-fonyl, $(C_1\text{-}C_6)$-alkylsulfinyl, $(C_6\text{-}C_{12})$-arylsulfonyl, or $(C_6\text{-}C_{12})$-arylsulfonyl which is substituted in the aryl radical by $(C_1\text{-}C_4)$-alkyl, $(C_1\text{-}C_4)$-alkoxy, halogen, $NO_2$, $NH_2$, CN or $CF_3$, or unsubstituted heteroarylcarbonyl or heteroarylsulfonyl in which the heteroaryl radicals contain one oxygen atom or 1 to 3 nitrogen atoms, or one of the substituents $R^6$, $R^7$ is hydrogen, as well as the

physiologically tolerated salts thereof, which comprises

a) reacting a compound of the formula II

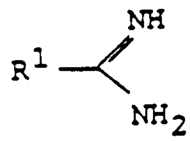

II

in which $R^1$ has the meanings indicated for formula I, or the acid addition salt thereof, with a compound of the formula III

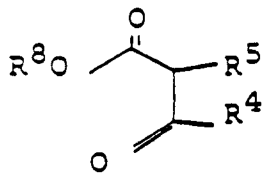

III

in which $R^4$ and $R^5$ have the meanings indicated for formula I, and $R^8$ denotes methyl or ethyl, or with a base salt thereof, to give a compound of the formula IV

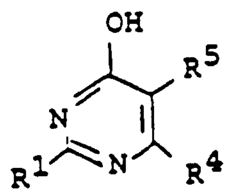

IV

in which $R^1$, $R^4$ and $R^5$ have the meanings indicated for formula I,

b) reacting a resulting compound IV with an inorganic acid chloride to give a pyrimidine derivative of the formula V

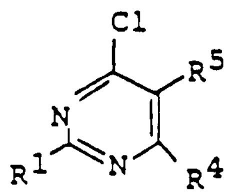

V

in which the radicals $R^1$, $R^4$ and $R^5$ have the meanings indicated for formula I,

c) reacting a resulting compound of the formula V with an amine of the formula VI

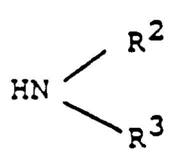

VI

in which $R^2$ and $R^3$ have the meanings indicated for formula I, to give a compound of the formula I, and

d) where appropriate introducing the radical(s) $R^6/R^7$ into a resulting compound of the formula I in which $R^2$ and $R^3$ form, together with the nitrogen atom carrying them, the piperazino, and

e) where appropriate converting a resulting compound of the formula I into a physiologically tolerated salt.

2. The process as claimed in claim 1, which comprises preparing a compound of the formula I, in formula I the substituents having the following meanings:

| | |
|---|---|
| $R^2$ and $R^5$ | (identical or different), hydrogen or $(C_1\text{-}C_6)$alkyl, |
| $R^4$ | hydrogen and |
| $R^2$, $R^3$ | together with the nitrogen to which they are bonded, a piperazine ring which is optionally substituted by identical or different groups $R^6$ and $R^7$, where |
| $R^6$, $R^7$ | denote $(C_1\text{-}C_6)$-alkyl, sulfamoyl, N-$(C_1\text{-}C_4)$-alkylsulfamoyl, N,N-$(C_1\text{-}C_4)$-dialkylsulfamoyl, $(C_1\text{-}C_6)$-alkoxycarbonyl, N,N-$(C_1\text{-}C_4)$-dialkylcarbamoyl, N-$(C_1\text{-}C_4)$-alkylcarbamoyl, carbamoyl, $(C_1\text{-}C_6)$-alkylcarbonyl, $(C_6\text{-}C_{12})$-arylcarbonyl, $(C_6\text{-}C_{12})$-arylcarbonyl which is substituted in the aryl radical by $(C_1\text{-}C_4)$-alkyl, $(C_1\text{-}C_4)$-alkoxy, halogen, $NO_2$, $NH_2$, CN or $CF_3$, or $(C_1\text{-}C_6)$-alkylsulfonyl, $(C_1\text{-}C_6)$-alkylsulfinyl, $(C_6\text{-}C_{12})$-arylsulfonyl, or $(C_6\text{-}C_{12})$-arylsulfonyl which is substituted in the aryl radical by $(C_1\text{-}C_4)$-alkyl, $(C_1\text{-}C_4)$-alkoxy, halogen, $NO_2$, $NH_2$, CN or $CF_3$, or unsubstituted heteroarylcarbonyl or heteroarylsulfonyl in which the heteroaryl radicals contain one oxygen atom or 1 to 3 nitrogen atoms, or one of the substituents $R^6$, $R^7$ is hydrogen, or the physiologically tolerated salts thereof. |

3. The process as claimed in claim 1, which comprises preparing a compound of the formula I, the substituents having the following meanings:

| | |
|---|---|
| $R^1$ and $R^5$ | (identical or different), hydrogen or $(C_1\text{-}C_4)$-alkyl, |
| $R^4$ | hydrogen and |
| $R^2$, $R^3$ | together with the nitrogen to which they are bonded, a piperazine ring which optionally carries in position 4 another substituent $R^6$, where |
| $R^6$ | denotes sulfamoyl, N-$(C_1\text{-}C_4)$-alkylsulfamoyl, N,N-$(C_1\text{-}C_4)$-dialkylsulfamoyl, carbamoyl, N-$(C_1\text{-}C_4)$-alkylcarbamoyl, N,N-$(C_1\text{-}C_4)$-dialkylcarbamoyl, $(C_1\text{-}C_6)$-alkylcarbonyl, $(C_6\text{-}C_{12})$-arylcarbonyl, or $(C_6\text{-}C_{12})$-arylcarbonyl which is substituted in the aryl radical by $(C_1\text{-}C_4)$-alkyl, $(C_1\text{-}C_4)$-alkoxy, halogen, $NO_2$, $NH_2$, CN or $CF_3$, or pyridinecarbonyl, or the physiologically tolerated salts thereof. |

4. The process as claimed in claim 1, which comprises preparing a compound of the formula I, wherein in formula I the substituents having the following meanings:

| | |
|---|---|
| $R^1$ | hydrogen or $(C_1\text{-}C_2)$-alkyl, |
| $R^4$ | hydrogen, |
| $R^5$ | hydrogen, |
| $R^2$, $R^3$ | form, together with the nitrogen to which they are bonded, a piperazine ring which optionally carries in position 4 another substituent $R^6$, where $R^6$ represents N-$(C_1\text{-}C_3)$-alkylsulfamoyl, N,N-$(C_1\text{-}C_2)$-dialkylsulfamoyl, N-$(C_1\text{-}C_2)$-alkylcarbamoyl, N,N-$(C_1\text{-}C_2)$-dialkylcarbamoyl, $(C_1\text{-}C_2)$alkylcarbonyl, phenylcarbonyl which is optionally substituted in the phenyl radical by $(C_1\text{-}C_2)$-alkyl, chlorine or $NO_2$, or pyridinecarbonyl, in particular N,N-dimethylsulfamoyl, phenylcarbonyl or pyridinecarbonyl, or the physiologically tolerated salts thereof. |

5. The process as claimed in claim 4, which comprises preparing a compound of the formula I, where $R^1$ is methyl, or the physiologically tolerated salts thereof.

6. The use of compounds of the formula I as in claim 1 and of the salts thereof as a tool in a pharmacological model.

**Revendications**

**Revendications pour les Etats contractants suivants : AT, BE, CH, DE, DK, FR, GB, GR, IT, LI, LU, NL, SE**

1. Dérivés de pyrimidine de formule I

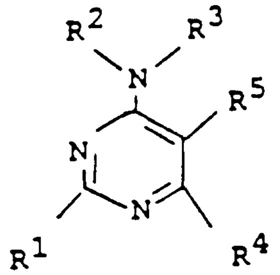

I

dans laquelle

$R^1$ et $R^5$ sont identiques ou différents et représentent un atome d'hydrogène ou un groupe alkyle en $C_1$-$C_6$,

$R^4$ est un atome d'hydrogène,

$R^2$ et $R^3$ forment ensemble, avec l'atome d'azote auquel ils sont liés, le groupe pipérazino ou un groupe pipérazino substitué par des groupes $R^6$ et $R^7$ identiques ou différents, $R^6$ et $R^7$ représentant un radical alkyle en $C_1$-$C_6$, sulfamoyle, N-alkyl($C_1$-$C_4$)-sulfamoyle, N,N-dialkyl($C_1$-$C_4$)-sulfamoyle, alcoxy($C_1$-$C_6$)-carbonyle, N,N-dialkyl($C_1$-$C_4$)-carbamoyle, N-alkyl($C_1$-$C_4$)-carbamoyle, N-aryl-($c_6$-$C_{12}$)-carbamoyle, un radical aryl($C_6$-$C_{12}$)-carbamoyle substitué sur le fragment aryle par un atome d'halogène ou par un groupe alkyle en $C_1$-$C_4$, alcoxy en $C_1$-$C_4$, $NO_2$, $NH_2$, CN ou $CF_3$, le radical carbamoyle, un radical alkyl-($C_1$-$C_6$)-carbonyle, aryl($c_6$-$C_{12}$)-carbonyle, un radical aryl($C_6$-$C_{12}$)-carbonyle substitué sur le fragment aryle par un atome d'halogène ou par un groupe alkyle en $C_1$-$C_4$,alcoxy en $C_1$-$C_4$, $NO_2$, $NH_2$, CN ou $CF_3$, un radical alkyl($C_1$-$C_6$)-sulfonyle, alkyl($C_1$-$C_6$)-sulfinyle, aryl($C_6$-$C_{12}$)-sulfonyle, un radical aryl($C_6$-$C_{12}$)-sulfonyle substitué sur le fragment aryle par un atome d'halogène ou par un groupe alkyle en $C_1$-$C_4$, alcoxy en $C_1$-$C_4$, $NO_2$, $NH_2$, CN ou $CF_3$, un radical hétéroarylcarbonyle ou hétéroarylsulfonyle non substitué, les radicaux hétéroaryle comportant un atome d'oxygène ou 1 à 3 atomes d'azote, ou l'un des substituants $R^6$, $R^7$ étant un atome d'hydrogène, et sels physiologiquement acceptables de ceux-ci.

2. Dérivés de pyrimidine selon la revendication 1 et leurs sels physiologiquement acceptables, caractérisés en ce que, dans la formule I, les substituants ont les significations suivantes:

$R^1$ et $R^5$ (identiques ou différents) représentent un atome d'hydrogène ou un groupe alkyle en $C_1$-$C_6$,

$R^4$ représente un atome d'hydrogène et

$R^2$ et $R^3$ conjointement avec l'atome d'azote auquel ils sont liés, forment un cycle pipérazine qui est éventuellement substitué par des groupes $R^6$ et $R^7$ identiques ou différents,

$R^6$ et $R^7$ représentent un groupe alkyle en $C_1$-$C_6$, sulfamoyle, N-alkyl($C_1$-$C_4$)-sulfamoyle, N,N-dialkyl($C_1$-$C_4$)-sulfamoyle, alcoxy($C_1$-$C_6$)-carbonyle, N,N-dialkyl($C_1$-$C_4$)-carbamoyle, N-alkyl($C_1$-$C_4$)-carbamoyle, carbamoyle, alkyl($C_1$-$C_6$)-carbonyle, aryl($C_6$-$C_{12}$)-carbonyle, un radical aryl($C_6$-$C_{12}$)-carbonyle substitué sur le fragment aryle par un atome d'halogène ou par un groupe alkyle en $C_1$-$C_4$, alcoxy en $C_1$-$C_4$, $NO_2$, $NH_2$, CN ou $CF_3$, un radical alkyl($C_1$-$C_6$)-sulfonyle, alkyl($C_1$-$C_6$)-sulfinyle, aryl($C_6$-$C_{12}$)-sulfonyle, un radical aryl($C_6$-$C_{12}$)-sulfonyle substitué sur le fragment aryle par un atome d'halogène ou par un groupe alkyle en $C_1$-$C_4$, alcoxy en $C_1$-$C_4$, $NO_2$, $NH_2$, CN ou $CF_3$, un radical hétéroarylcarbonyle ou hétéroarylsulfonyle non substitué, les radicaux hétéroaryle comportant un atome d'oxygène ou 1 à 3 atomes d'azote, ou l'un des substituants $R^6$ et $R^7$ étant un atome d'hydrogène.

3. Dérivés de pyrimidine selon la revendication 1 et leurs sels physiologiquement acceptables, caractérisés en ce que, dans la formule I, les substituants ont les significations suivantes:

$R^1$ et $R^5$ (identiques ou différents), représentent un atome d'hydrogène ou un radical alkyle en $C_1$-$C_4$,

$R^4$ représente un atome d'hydrogène et

$R^2$ et $R^3$ forment ensemble, avec l'atome d'azote auquel ils sont liés, un cycle pipérazine qui porte éventuellement en position 4 un autre substituant $R^6$ ,

$R^6$ représentant un radical sulfamoyle, N-alkyl($C_1$-$C_4$)-sulfamoyle, N,N-dialkyl($C_1$-$C_4$)-sulfamoyle, carbamoyle, N-alkyl($C_1$-$C_4$)-carbamoyle, N,N-dialkyl($C_1$-$C_4$)-carbamoyle, alkyli ($C_1$-$C_6$)-carbonyle un radical aryl($C_6$-$C_{12}$)-carbonyle, un radical aryl($C_6$-$C_{12}$)-carbonyle substitué sur le fragment aryle par un atome d'halogène ou par un groupe alkyle en $C_1$-$C_4$, alcoxy en $C_1$-$C_4$, $NO_2$, $NH_2$, CN ou $CF_3$, ou le radical pyridinecarbonyle.

4. Dérivés de pyrimidine selon la revendication 1 et leurs sels physiologiquement acceptables, caractérisés en ce que, dans la formule I, les substituants ont les significations suivantes:

$R^1$ représente un atome d'hydrogène ou un radical alkyle en $C_1$-$C_2$,

$R^4$ représente un atome d'hydrogène,

$R^5$ représente un atome d'hydrogène,

$R^2$ et $R^3$ forment, conjointement avec l'atome d'azote auquel ils sont liés, un cycle pipérazine qui porte éventuellement en position 4 un autre substituant $R^6$, $R^6$ étant un radical N-alkyl-($C_1$-$C_3$)-sulfamoyle, N,N-dialkyl($C_1$-$C_2$)-sulfamoyle, N-alkyl($C_1$-$C_2$)-carbamoyle, N,N-dialkyl($C_1$-$C_2$)-carbamoyle, alkyl($C_1$-$C_2$)-carbonyle, un radical phénylcarbonyle qui est éventuellement substitué sur le fragment phényle par un atome de chlore ou par un groupe alkyle en $C_1$-$C_2$ ou $NO_2$, ou le radical pyridinecarbonyle, en particulier le radical N,N-diméthylsulfamoyle, phénylcarbonyle ou pyridinecarbonyle.

5. Dérivés de pyrimidine selon la revendication 4 et leurs sels physiologiquement acceptables, caractérisés en ce que, dans la formule I, $R^1$ représente le groupe méthyle.

6. Procédé pour la préparation des composés de formule I selon la revendication 1, caractérisé en ce que, d'une façon connue en soi,

a) on fait réagir un composé de formule II

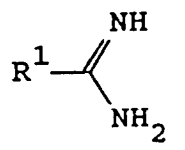

II

dans laquelle $R^1$ a les significations données à propos de la formule I, ou un de ses sels d'addition avec un acide, avec un composé de formule III

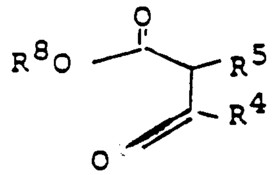

III

dans laquelle $R^4$ et $R^5$ ont les significations données à propos de la formule I, et $R^8$ représente le groupe méthyle ou éthyle, ou avec un de ses sels d'addition avec une base, pour aboutir à un composé de formule IV

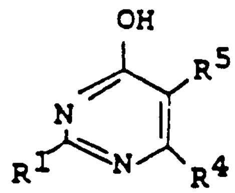

IV

dans laquelle $R^1$, $R^4$ et $R^5$ ont les significations données à propos de la formule I,
b) on fait réagir un composé IV obtenu avec un chlorure d'acide minéral, pour aboutir à un dérivé de pyrimidine de formule V

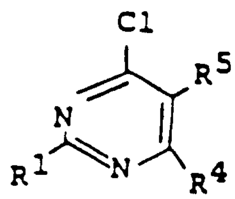

V

dans laquelle les radicaux $R^1$, $R^4$ et $R^5$ ont les significations données à propos de la formule I,
c) on fait réagir un composé de formule V obtenu avec une amine de formule VI

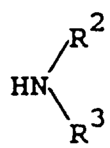

VI

dans laquelle $R^2$ et $R^3$ ont les significations données à propos de la formule I, pour aboutir à un composé de formule I, et
d) éventuellement, dans un composé de formule I obtenu, dans lequel $R^2$ et $R^3$ forment, conjointement avec l'atome d'azote qui les porte, le radical pipérazino, on introduit le ou les radicaux $R^6$/$R^7$, et
e) éventuellement on convertit un composé de formule I obtenu en un sel physiologiquement acceptable.

**7.** Utilisation des composés de formule I selon la revendication 1 et de leurs sels en tant que moyen dans un modèle pharmacologique.

**Revendications pour l'Etat contractant suivant : ES**

**1.** Procédé pour la préparation de dérivés de pyrimidine de formule I

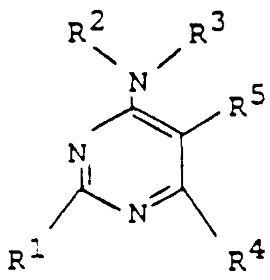

I

dans laquelle
$R^1$ et $R^5$ sont identiques ou différents et représentent un atome d'hydrogène ou un groupe alkyle

en $C_1$-$C_6$,

$R^4$ est un atome d'hydrogène,

$R^2$ et $R^3$ forment ensemble, avec l'atome d'azote auquel ils sont liés, le groupe pipérazino ou un groupe pipérazino substitué par des groupes $R^6$ et $R^7$ identiques ou différents, $R^6$ et $R^7$ représentant un radical alkyle en $C_1$-$C_6$, sulfamoyle, N-alkyl($C_1$-$C_4$)-sulfamoyle, N,N-dialkyl($C_1$-$C_4$)-sulfamoyle, alcoxy($C_1$-$C_6$)-carbonyle, N,N-dialkyl($C_1$-$C_4$)-carbamoyle, N-alkyl($C_1$-$C_4$)-carbamoyle, N-aryl($C_6$-$C_{12}$)-carbamoyle, un radical aryl($C_6$-$C_{12}$)-carbamoyle substitué sur le fragment aryle par un atome d'halogène ou par un groupe alkyle en $C_1$-$C_4$, alcoxy en $C_1$-$C_4$, $NO_2$, $NH_2$, CN ou $CF_3$, le radical carbamoyle, un radical alkyl-($C_1$-$C_6$)-carbonyle, aryl($C_6$-$C_{12}$)-carbonyle, un radical aryl($C_6$-$C_{12}$)-carbonyle substitué sur le fragment aryle par un atome d'halogène ou par un groupe alkyle en $C_1$-$C_4$,alcoxy en $C_1$-$C_4$, $NO_2$, $NH_2$, CN ou $CF_3$, un radical alkyl($C_1$-$C_6$)-sulfonyle, alkyl($C_1$-$C_6$)-sulfinyle, aryl($C_6$-$C_{12}$)sulfonyle, un radical aryl($C_6$-$C_{12}$)-sulfonyle substitué sur le fragment aryle par un atome d'halogène ou par un groupe alkyle en $C_1$-$C_4$, alcoxy en $C_1$-$C_4$, $NO_2$, $NH_2$, CN ou $CF_3$, un radical hétéroarylcarbonyle ou hétéroarylsulfonyle non substitué, les radicaux hétéroaryle comportant un atome d'oxygène ou 1 à 3 atomes d'azote, ou l'un des substituants $R^6$, $R^7$ étant un atome d'hydrogène,

ainsi que de leurs sels physiologiquement acceptables, caractérisé en ce que

a) on fait réagir un composé de formule II

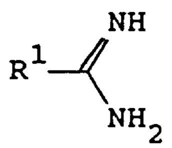

II

dans laquelle $R^1$ a les significations données à propos de la formule I, ou un de ses sels d'addition avec un acide, avec un composé de formule III

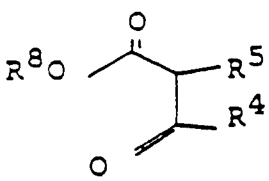

III

dans laquelle $R^4$ et $R^5$ ont les significations données à propos de la formule I, et $R^8$ représente le groupe méthyle ou éthyle, ou avec un de ses sels d'addition avec une base, pour aboutir à un composé de formule IV

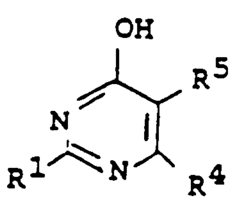

IV

dans laquelle $R^1$, $R^4$ et $R^5$ ont les significations données à propos de la formule I,

b) on fait réagir un composé IV obtenu avec un chlorure d'acide minéral, pour aboutir à un dérivé de pyrimidine de formule V

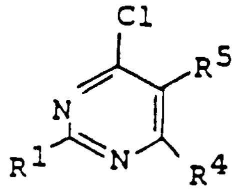

V

dans laquelle les radicaux $R^1$ $R^4$ et $R^5$ ont les significations données à propos de la formule I,
c) on fait réagir un composé de formule V ainsi obtenu avec une amine de formule VI

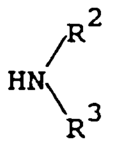

VI

dans laquelle $R^2$ et $R^3$ ont les significations données à propos de la formule I, pour aboutir à un composé de formule I, et
d) éventuellement, dans un composé de formule I obtenu, dans lequel $R^2$ et $R^3$ forment, conjointement avec l'atome d'azote qui les porte, le radical pipérazino, on introduit le ou les radicaux $R^6$/$R^7$, et
e) éventuellement on convertit un composé de formule I obtenu en un sel physiologiquement acceptable.

**2.** Procédé selon la revendication 1, caractérisé en ce que l'on prépare un composé de formule I, dans la formule I, les substituants ayant les significations suivantes:

$R^1$ et $R^5$ (identiques ou différents) représentent un atome d'hydrogène ou un groupe alkyle en $C_1$-$C_6$,

$R^4$ représente un atome d'hydrogène et

$R^2$ et $R^3$, conjointement avec l'atome d'azote auquel ils sont liés, forment un cycle pipérazine qui est éventuellement substitué par des groupes $R^6$ et $R^7$ identiques ou différents,

$R^6$ et $R^7$ représentent un groupe alkyle en $C_1$-$C_6$, sulfamoyle, N-alkyl($C_1$-$C_4$)-sulfamoyle, N,N-dialkyl($C_1$-$C_4$)-sulfamoyle, alcoxy($C_1$-$C_6$)-carbonyle, N,N-dialkyl($C_1$-$C_4$)-carbamoyle, N-alkyl($C_1$-$C_4$)-carbamoyle, carbamoyle, alkyl($C_1$-$C_6$)-carbonyle, aryl($C_6$-$C_{12}$)-carbonyle, un radical aryl($C_6$-$C_{12}$)-carbonyle substitué sur le fragment aryle par un atome d'halogène ou par un groupe alkyle en $C_1$-$C_4$, alcoxy en $C_1$-$C_4$, $NO_2$, $NH_2$, CN ou $CF_3$, un radical alkyl($C_1$-$C_6$)-sulfonyle, alkyl($C_1$-$C_6$)-sulfinyle, aryl($C_6$-$C_{12}$)-sulfonyle, un radical aryl($C_6$-$C_{12}$)-sulfonyle substitué sur le fragment aryle par un atome d'halogène ou par un groupe alkyle en $C_1$-$C_4$, alcoxy en $C_1$-$C_4$, $NO_2$, $NH_2$, CN ou $CF_3$, un radical hétéroarylcarbonyle ou hétéroarylsulfonyle non substitué, les radicaux hétéroaryle comportant un atome d'oxygène ou 1 à 3 atomes d'azote, ou l'un des substituants $R^6$ et $R^7$ étant un atome d'hydrogène on les sels plupiologique ment acceptable.

**3.** Procédé selon la revendication 1, caractérisé en ce que l'on prépare un composé de formule I dans laquelle les substituants ont les significations suivantes:

$R^1$ et $R^5$ (identiques ou différents), représentent un atome d'hydrogène ou un radical alkyle en $C_1$-$C_4$,

$R^4$ représente un atome d'hydrogène et

$R^2$ et $R^3$ forment ensemble, avec l'atome d'azote auquel ils sont liés, un cycle pipérazine qui porte éventuellement en position 4 un autre substituant $R^6$,

$R^6$ représentant un radical sulfamoyle, N-alkyl($C_1$-$C_4$)-sulfamoyle, N,N-dialkyl($C_1$-$C_4$)-sulfamoyle, carbamoyle, N-alkyl($C_1$-$C_4$)-carbamoyle, N,N-dialkyl($C_1$-$C_4$)-carbamoyle, alkyl-($C_1$-$C_6$)-carbonyle, aryl($C_6$-$C_{12}$)-carbonyle, un radical aryle ( $C_6$ -$C_{12}$ )-carbonyle substitué sur le fragment aryle par un atome d'halogène ou par un groupe alkyle en $C_1$-$C_4$, alcoxy en $C_1$-$C_4$, $NO_2$, $NH_2$, CN ou $CF_3$, ou le radical pyridinecarbonyle,, on les sels

plupiologique ment acceptable.

4. Procédé selon la revendication 1, caractérisé en ce que l'on prépare un composé de formule I, dans la formule I, les substituants ont les significations suivantes:
   - $R^1$ représente un atome d'hydrogène ou un radical alkyle en $C_1$-$C_2$,
   - $R^4$ représente un atome d'hydrogène,
   - $R^5$ représente un atome d'hydrogène,
   - $R^2$ et $R^3$ forment, conjointement avec l'atome d'azote auquel ils sont liés, un cycle pipérazine qui porte éventuellement en position 4 un autre substituant $R^6$, $R^6$ étant un radical N-alkyl-($C_1$-$C_3$)-sulfamoyle, N,N-dialkyl($C_1$-$C_2$)-sulfamoyle, N-alkyl($C_1$-$C_2$)-carbamoyle, N,N-dialkyl($C_1$-$C_2$)-carbamoyle, alkyl($C_1$-$C_2$)-carbonyle, un radical phénylcarbonyle qui est éventuellement substitué sur le fragment phényle par un atome de chlore ou par un groupe alkyle en $C_1$-$C_2$ ou $NO_2$, ou le radical pyridinecarbonyle, en particulier le radical N,N-diméthylsulfamoyle, phénylcarbonyle ou pyridinecarbonyle , on les sels plupiologique ment acceptable.

5. Procédé selon la revendication 4, caractérisé en ce que l'on prépare un composé de formule I dans lequel $R^1$ représente le groupe méthyle, ou ses sels physiologiquement acceptables.

6. Utilisation des composés de formule I selon la revendication 1 et de leurs sels en tant que moyen dans un modèle pharmacologique.